(19) Europäisches Patentamt
European Patent Office
Office européen des brevets

(11) **EP 2 357 020 B1**

(12) **FASCICULE DE BREVET EUROPEEN**

(45) Date de publication et mention
de la délivrance du brevet:
**29.08.2012 Bulletin 2012/35**

(51) Int Cl.:
*A61N 1/362* *(2006.01)*  *A61N 1/365* *(2006.01)*
*A61N 1/368* *(2006.01)*

(21) Numéro de dépôt: **10194984.0**

(22) Date de dépôt: **14.12.2010**

(54) **Dispositif médical actif de type stimulateur cardiaque, défibrillateur et/ou resynchroniseur, à optimisation automatique du délai atrioventriculaire**

Aktive medizinische Vorrichtung vom Typ Herzschrittmacher, Defibrillator und/oder Resynchronisator mit automatischer Optimierung der atrioventrikulären Verzögerung

Active medical device of the pacemaker, defibrillator or resynchronizer type, with automatic atrioventricular delay optimisation

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.02.2010 FR 1051120**

(43) Date de publication de la demande:
**17.08.2011 Bulletin 2011/33**

(73) Titulaire: **Sorin CRM SAS**
**92143 Clamart Cedex (FR)**

(72) Inventeur: **Makdissi, Alaa**
**75011, PARIS (FR)**

(74) Mandataire: **Dupuis-Latour, Dominique et al**
**Bardehle Pagenberg**
**10, boulevard Haussmann**
**75009 Paris (FR)**

(56) Documents cités:
**EP-A1- 2 070 562    US-A1- 2007 179 542**
**US-B1- 6 522 923**

• **DUPUIS J.M ET AL: "Programming optimal atrioventricular delay in dual chamber pacing using peak endocardial acceleration: comparison with a standard echocardiographic procedure", PACE, vol. 26, janvier 2003 (2003-01), pages 210-213, XP002596976,**

EP 2 357 020 B1

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de détection d'un trouble du rythme.

Elle concerne plus précisément les implants mettant en oeuvre un mode de fonctionnement "double chambre", avec détection auriculaire et ventriculaire, et stimulation ventriculaire. Dans ce mode "double chambre", le dispositif surveille l'activité ventriculaire après un événement auriculaire spontané (détection d'une onde P de dépolarisation auriculaire) ou stimulé (application d'une impulsion A de stimulation auriculaire) et, en même temps, commence à compter un délai dit "délai atrioventriculaire" ou "délai auriculo-ventriculaire" (DAV ou AVD). Si aucune activité spontanée ventriculaire (onde R) n'a été détectée à l'issue de ce DAV, alors le dispositif déclenche une stimulation de ce ventricule (application d'une impulsion V).

Il est nécessaire de réévaluer régulièrement les réglages du dispositif pour les réajuster éventuellement : en effet, les effets bénéfiques procurés par la thérapie peuvent conduire, à terme, à modifier la configuration primitive et le paramétrage de la stimulation.

Pour l'ajustement de ces paramètres de stimulation, et notamment du DAV, la technique de référence est l'évaluation par échocardiographie avec estimation des délais caractéristiques de la systole, en particulier du temps d'ouverture de la valve aortique.

Cette procédure doit toutefois être mise en oeuvre en milieu hospitalier et par un personnel qualifié, elle est longue et coûteuse et ne peut pas être appliquée aussi souvent que cela serait utile ou nécessaire sans interférer avec la vie quotidienne du patient.

De plus, dans le cas d'un dispositif "multisite", comprenant plusieurs sites de stimulation, une autre difficulté tient au fait que l'évaluation échocardiographique nécessite de tester successivement plusieurs configurations de stimulation (sélection de sites différents et/ou séquencement différent des impulsions appliquées aux sites sélectionnés), en déterminant pour chacune de ces configurations un DAV optimal. Le nombre de combinaisons à tester est de ce fait important, et implique une procédure longue et difficile à gérer, ce qui exclut d'en faire une opération de routine.

Cet inconvénient est typique des dispositifs permettant d'assurer une stimulation conjointe et permanente des ventricules gauche et droit afin de resynchroniser ces derniers, technique dite "CRT" (*Cardiac Resynchronization Therapy*) ou "BVP" (*Bi-Ventricular Pacing*). En effet, outre le réglage du DAV, il est nécessaire d'optimiser le délai dit "délai interventriculaire" (DVV ou VVD) séparant les instants respectifs de stimulation des ventricules gauche et droit, qui doit être ajusté de manière à resynchroniser la contraction des ventricules avec optimisation fine de l'état hémodynamique du patient. La recherche du couple {DAV, DVV} optimal peut de ce fait être très longue, car elle requiert de multiples balayages du DAV, pour les diverses valeurs de DVV testées séparément.

Le EP 2 070 562 A1(ELA Medical) décrit un tel dispositif de stimulation biventriculaire prévoyant de tester par balayages successifs une pluralité de configurations de stimulation.

Il subsiste de ce fait un besoin réel de disposer d'une technique permettant d'évaluer de façon simple, rapide, automatisée et précise l'incidence des différents paramètres de la stimulation, notamment du DAV, de manière à pouvoir optimiser l'état hémodynamique du patient.

Une méthode automatisée d'optimisation du DAV est décrite dans JM Dupuis et al. : Programming Optimal Atrioventricular Delay in Dual Chamber Pacing Using Peak Endocardial Acceleration : Comparison with a Standard Echocardiographic Procedure, PACE 2003; 26: [Pt. II], 210-213. Cette technique consiste à exécuter un balayage du DAV dans une configuration de stimulation donnée, pour tracer une caractéristique donnant la valeur du pic d'accélération endocardiaque PEA (*Peak Endocardial Acceleration*) en fonction du DAV. La valeur considérée optimale du DAV est celle du point d'inflexion de la caractéristique, point correspondant à une durée de remplissage maximale du ventricule sans troncature de l'onde A (retard minimal entre la fermeture de la valve mitrale et le début du complexe QRS).

L'algorithme correspondant, s'il donne des résultats satisfaisants, requiert toutefois plusieurs minutes, tout particulièrement dans le cas d'un dispositif multisite ou d'un dispositif CRT, toujours en raison des multiples balayages de DAV pour les diverses valeurs des autres paramètres testés séparément (notamment le DVV), avant de pouvoir sélectionner le couple {DAV, DVV} optimal.

Une autre technique d'optimisation, beaucoup plus rapide et pouvant être mise en oeuvre en temps réel, est exposée dans les WO 2006/090397 A2 et WO 2006/126185 A2. L'algorithme décrit dans ces documents utilise un réseau neuronal de type *spike* pour identifier le maximum d'une fonction hémodynamique (*stroke volume*).

Un réseau *spike* nécessite toutefois un processeur dédié, impliquant donc la conception d'un implant spécifique plus complexe, avec une consommation électrique supérieure. Une version logicielle de cet algorithme est certes possible, mais exige dans ce cas des ressources informatiques qui ne sont pas à la portée des microcontrôleurs à ultra-faible consommation tel que ceux utilisés dans les implants cardiaques.

Le WO 2008/010220 décrit une technique du même type, où le processeur neuronal *spike* est combiné à un algorithme d'apprentissage renforcé (*Q-learning*), qui apprend et associe les conditions cardiaques aux délais optimaux. L'utilisation de cet apprentissage renforcé permet d'accroître l'immunité aux bruits et d'augmenter la vitesse de convergence de l'algorithme d'asservissement.

Toutefois, s'il permet d'atteindre d'excellentes performances, cet apprentissage renforcé nécessite des ressources matérielles dédiées supplémentaires, notamment l'ajout d'un microcontrôleur en plus du processeur neuronal *spike.*

L'invention a pour but de remédier aux inconvénients exposés ci-dessus, en proposant une nouvelle technique simple, rapide, automatisée et fiable pour optimiser le paramètre DAV.

On verra en particulier que la technique de l'invention peut être appliquée quasiment en temps réel, c'est-à-dire avec un temps de réponse de quelques cycles cardiaques seulement, et en ne mettant en oeuvre que des moyens matériels et logiciels simples, tout à fait compatibles avec ce qui est disponible au sein d'un implant cardiaque actuel.

À cet effet, l'invention propose un dispositif médical actif d'un type connu, par exemple d'après le EP 2 070 562 A1 précité, comprenant les éléments énoncés dans le préambule de la revendication 1. Les éléments propres à l'invention sont énoncés dans la partie caractérisante de la revendication 1. Les sous-revendications visent des mises en oeuvre particulières, avantageuses.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est un synoptique montrant les divers éléments impliqués dans un asservissement hémodynamique temps réel, appliqué au cas particulier d'un dispositif CRT.

La Figure 2 illustre la variation, en fonction du DAV, de la valeur du pic d'accélération endocardiaque PEA obtenu à partir d'un capteur hémodynamique.

La Figure 3 illustre schématiquement, en rapport avec la caractéristique PEA/DAV, les valeurs successivement recueillies du signal hémodynamique, et la manière d'en déduire une valeur de DAV optimale.

La Figure 4 est un organigramme expliquant l'algorithme de détection d'un changement d'état hémodynamique du patient, du repos vers l'effort et *vice versa*.

La Figure 5 montre la manière dont évoluent au cours de cycles cardiaques successifs la fréquence cardiaque, la moyenne à long terme et la moyenne à court terme de cette fréquence cardiaque, ainsi que l'écart entre ces deux moyennes par rapport à un seuil de détection d'état d'effort.

On va maintenant décrire un exemple de réalisation de l'invention.

Cet exemple sera décrit dans le cadre d'un dispositif CRT, c'est-à-dire un dispositif resynchroniseur permettant d'assurer une stimulation conjointe et permanente des deux ventricules afin de resynchroniser ces derniers. L'invention s'applique en effet de façon très avantageuse aux dispositifs de ce type, qui nécessitent l'ajustement conjoint de plusieurs paramètres interdépendants, notamment les délais DAV et DVV.

L'invention n'est toutefois pas limitée aux dispositifs de type CRT, et elle s'applique comme on le comprendra à tous les dispositifs pour lesquels il est nécessaire d'optimiser la valeur du DAV au repos ou à l'effort, c'est-à-dire de façon générale tous les dispositif de type "double chambre", en incluant bien entendu les dispositifs plus complexes (triple chambre, quadruple chambre, multisite, CRT, etc.) ainsi que les défibrillateurs intégrant des fonctions de stimulation.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym ,* notamment *Paradym CRT,* produits et commercialisés par Sorin CRM, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

*Configuration générale du dispositif*

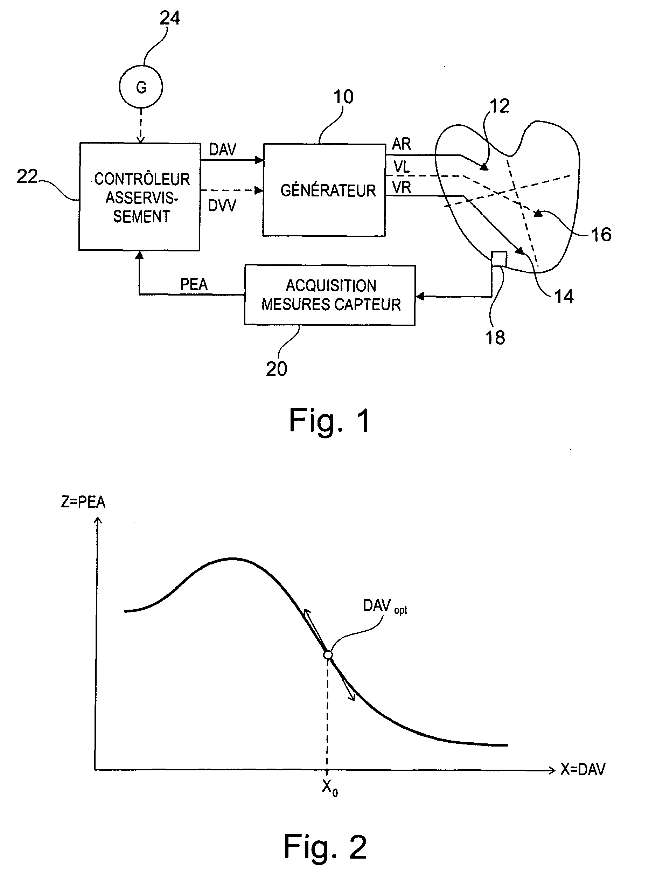

On a illustré sur la Figure 1 de façon schématique, sous forme de blocs fonctionnels, les différents éléments impliqués dans le contrôle du DAV (et du DVV, dans le cas d'un dispositif CRT).

La référence 10 désigne le générateur du dispositif, qui est interfacé avec le coeur au moyen de sondes permettant de détecter les signaux de dépolarisation du myocarde et stimuler celui-ci par délivrance d'impulsions aux différentes cavités : une sonde 12 implantée dans l'oreillette droite (AR) et une sonde 14 implantée dans le ventricule droit (VR) permettent d'optimiser le délai atrioventriculaire DAV entre les instants de stimulation de l'oreillette et du ventricule droit, tandis que (pour un dispositif CRT) une sonde 16 implantée au voisinage du ventricule gauche (VL) permet, en combinaison avec la sonde 14 implantée dans le ventricule droit, d'optimiser le délai interventriculaire DVV entre les ventricules gauche et droit. Par ailleurs, un capteur hémodynamique 18 mesure une quantité corrélée avec le débit cardiaque. Un capteur hémodynamique est un capteur permettant d'estimer les variations de contractilité corrélées aux augmentations de la pression sanguine. Il se distingue des capteurs d'activité (capteurs d'accélération par exemple) et des capteurs métaboliques (capteurs de ventilation-minute par exemple), qui ne sont destinés qu'à diagnostiquer la présence ou non d'un exercice par le patient et à quantifier ses besoins métaboliques, par exemple pour adapter la fréquence cardiaque de stimulation en fonction du niveau détecté. Un capteur hémodynamique peut en revanche non seulement assurer un suivi des efforts du patient comme un capteur d'activité ou un capteur métabolique, mais également donner une indication de la tolérance hémodynamique du patient par rapport à certains événements, en particulier la tolérance à une modification des paramètres DAV (et DVV, le cas échéant) par le dispositif.

L'invention concerne le cas où le capteur hémodynamique 18 est un capteur d'accélération endocardiaque dit "capteur PEA" (*Peak Endocardial Acceleration*).

Pour diverses descriptions d'un tel capteur PEA, on pourra se référer notamment au EP 0 515 319 A1 (Sorin Biomedica Cardio SpA), qui décrit la manière de recueillir un signal d'accélération endocardiaque (signal EA) au moyen d'une sonde endocavitaire pourvue d'une électrode distale de stimulation implantée au fond du ventricule et intégrant un micro-accéléromètre permettant de mesurer l'accélération endocardiaque, et au EP 0 655 260 A1 (Sorin Biomedica Cardio SpA), qui décrit une manière de traiter le signal d'accélération endocardiaque mesuré pour en dériver notamment la valeur des pics d'accélération endocardiaque correspondant aux deux bruits majeurs qu'il est possible de reconnaître dans chaque cycle d'un coeur sain.

De tels capteurs ont été développés par la Demanderesse notamment sous la référence *SonR Fix* (marque déposée) pour la gamme de stimulateurs *Paradym* et *Paradym CRT.*

L'invention peut être également mise en oeuvre dans une configuration où le signal PEA est obtenu de manière non invasive au moyen d'un capteur externe au lieu d'un capteur implanté, par exemple au moyen d'un capteur accélérométrique fixé sur la poitrine du patient au niveau du sternum. Qu'il s'agisse d'un capteur implanté ou externe, le capteur 18 délivre un signal corrélé avec le débit cardiaque, qui est transmis à un circuit d'acquisition des mesures 20 (ce circuit pouvant être incorporé au générateur du dispositif implanté, ou bien situé à l'extérieur du corps du patient).

Le circuit 20 délivre un signal hémodynamique PEA, qui est transmis à une unité 22 formant contrôleur d'asservissement. Cette transmission peut être directe (dans le cas d'un traitement purement interne à l'implant), ou bien être opérée par télémétrie (dans le cas d'un capteur externe et d'un contrôleur 22 intégré à l'implant ou, inversement, dans le cas d'un capteur implanté et d'un contrôleur 22 externe, par exemple un contrôleur intégré à un programmateur utilisé pour paramétrer le générateur lors d'une visite chez un praticien).

Le contrôleur 22 met en oeuvre un algorithme d'asservissement permettant d'obtenir les valeurs optimales recherchées des paramètres de pilotage du générateur, notamment le DAV optimal $DAV_{opt}$ (ainsi que le DVV optimal, pour un dispositif CRT). L'ensemble peut également comprendre (mais non nécessairement) un capteur auxiliaire 24 de mesure d'un paramètre à prépondérance physique tel qu'un accéléromètre ou "capteur G" permettant de détecter rapidement un début ou une fin d'activité du patient.

*Algorithme d'optimisation du DAV*

Le circuit d'acquisition des mesures 20 délivre une information représentative du pic d'accélération endocardiaque PEA, plus précisément, du premier pic d'accélération endocardiaque ("PEA1") qui correspond à la fermeture des valvules mitrale et tricuspide, au début de la phase de contraction ventriculaire isovolumique (systole), dont les variations sont étroitement liés aux variations de la pression dans le ventricule et peuvent donc constituer un paramètre représentatif de la contractilité du myocarde. L'amplitude du pic PEA1 est notamment corrélée au maximum positif de la variation de pression dP/dt dans le ventricule gauche.

Les variations de ce signal en fonction du DAV suivent une loi Z = f(DAV) représentée par la caractéristique illustrée Figure 2.

La valeur optimale du DAV correspond au point $DAV_{opt}$ situé au point d'inflexion de cette caractéristique, point correspondant à une durée de remplissage maximale du ventricule sans troncature de l'onde A (retard minimal entre la fermeture de la valve mitrale et le début du complexe QRS). Au voisinage de ce point d'inflexion représentatif de l'optimum du DAV, la caractéristique peut être approximée par une fonction sigmoïde, c'est-à-dire une fonction contenant des termes exponentiels : si l'on désigne *X* la valeur du DAV courant et $X_0$ la valeur du DAV optimum, la forme d'une courbe sigmoïde reflétant les variations du signal représentatif de la valeur du PEA au voisinage de l'optimum recherché correspondra à une fonction du type :

$$Y = a + b\left(\frac{1}{1 + \exp(c(X - X_0))}\right)$$

où les paramètres *a, b, c* et $X_0$ de la sigmoïde sont des quantités pouvant varier au cours du temps et selon l'état du patient (repos ou effort).

Pour optimiser le DAV, on se base sur le fait que le point $X_0$, qui représente un point d'inflexion de la sigmoïde, correspond à une annulation de la dérivée seconde de la fonction $Y$.

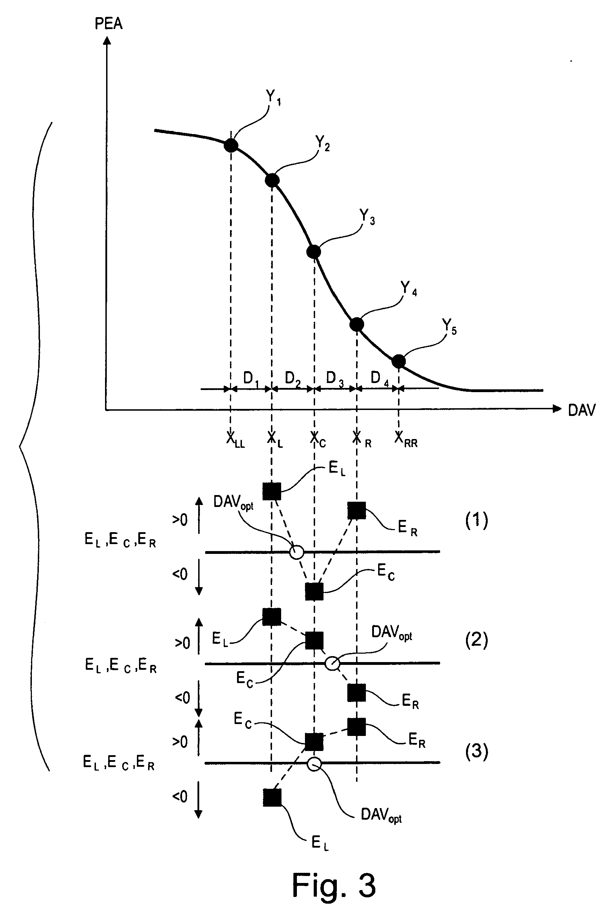

Le détail de l'algorithme d'optimisation selon l'invention va maintenant être expliqué, en référence à la Figure 3.

Le point de départ de cet algorithme consiste à partir de la dernière valeur optimale du DAV, ci-après désignée $X_C$ (valeur centrale de la valeur courante X du DAV) et de rechercher le point pour lequel la dérivée seconde est nulle, ou minimale, autour de cette valeur $X_C$. (lorsqu'aucune valeur de DAV optimal n'est connue, notamment au démarrage de l'algorithme, on choisit une valeur moyenne typique, par exemple $X_C = 120$ ms).

Pour ce faire, l'algorithme va tester plusieurs points entourant ce délai central $X_C$ et, à partir des résultats de ces tests, déterminera par une interpolation ou par une recherche de minimum la valeur du DAV optimal courante.

En d'autres termes, au lieu d'effectuer un balayage continu de toutes les valeurs du DAV pour procéder à l'optimisation, ce balayage est remplacé par des tests effectués sur un nombre limité de valeurs discrètes du DAV, à partir desquels l'algorithme déduit la position de l'optimum directement à partir des résultats de ces tests, et indépendamment de l'ordre dans lequel ils ont été effectués (à la différence d'un balayage, qui présuppose une variation continue du DAV sur une plage relativement étendue). Outre le point central $X_C$ correspondant au dernier DAV optimal connu, on va opérer des mesures pour deux points $X_L$ et $X_{LL}$ situés à gauche du point $X_C$, et deux points $X_R$ et $X_{RR}$ situés à la droite de ce même point $X_C$ de la caractéristique.

Ces divers points $X_{LL}$, $X_L$, $X_C$, $X_R$ et $X_{RR}$ sont séparés par des délais correspondants $D_1$, $D_2$, $D_3$ et $D_4$, de préférence égaux entre eux et correspondant au pas D de balayage du DAV, typiquement $D = 20$ ms.

Par rapport au délai central $X_C$, les valeurs des délais $X_L$, $X_R$, $X_{LL}$ et $X_{RR}$ sont données par :

$$X_L = X_C - D \text{ (DAV à gauche)}$$
$$X_R = X_C + D \text{ (DAV à Droite)}$$
$$X_{LL} = X_L - D$$
$$X_{RR} = X_R + D$$

S'il n'est pas possible de choisir un pas fixe D, les cinq points choisis seront tels que :

$$X_L = X_C - D_2$$
$$X_R = X_C + D_3$$
$$X_{LL} = X_L - D_1$$
$$X_{RR} = X_R + D_4$$

Soient $Y_1$, $Y_2$, $Y_3$, $Y_4$ et $Y_5$ les 5 mesures de PEA qui correspondent aux valeurs respectives $X_{LL}$, $X_L$, $X_C$, $X_R$, $X_{RR}$ du DAV (chaque valeur $Y_1$ à $Y_5$ étant de préférence obtenue en moyennant quatre à dix mesures du PEA).

L'algorithme calcule alors :

- la dérivée seconde au centre $E_C$, au point $(X_C, Y_3)$,
- la dérivée seconde à gauche $E_L$, au point $(X_L, Y_2)$, et
- la dérivée seconde à droite $E_R$, au point $(X_R, Y_4)$,

ce qui donne :

$$E_C = 2*((D_2+D_3)*Y_3-D_3*Y_2-D_1*Y_4)/(D_2+D_3)$$
$$E_L = 2*((D_1+D_2)*Y_2-D_2*Y_1-D_1*Y_3)/(D_1+D_2)$$
$$E_R = 2*((D_3+D_4)*Y_4-D_3*Y_3-D_1*Y_5)/(D_3+D_4)$$

Quand le pas est fixe, le calcul est simplifié en :

$$E_C = 2*Y_3-Y_2-Y_4$$
$$E_L = 2*Y_2-Y_1-Y_3$$
$$E_R = 2*Y_4-Y_3-Y_5$$

On notera que, les valeurs X et Y étant des valeurs échantillonnées numérisées, le calcul de dérivée seconde se réduit à un calcul d'accroissement, ne requérant que des opérations arithmétiques simples.

Une fois les trois dérivées secondes $E_L$, $E_C$ et $E_R$ calculées, on recherche le DAV qui correspond à un passage par zéro de la dérivée seconde, par application des trois règles (1), (2) et (3) ci-après :

Si (($E_L > 0$) et ($E_C < 0$)) alors
*Règle (1)*
On calcule le passage par zéro par interpolation linéaire :

$$DAV_{opt} = X_L + E_L*(X_C - X_L)/(E_L - E_C)$$
Optimum trouvé → Sortie de l'algorithme

Si (($E_C > 0$) et ($E_R < 0$)) alors
*Règle (2)*
On calcule le passage par zéro par interpolation linéaire :

$$DAV_{opt} = X_C + E_C*(X_R - X_C)/(E_C - E_R)$$
Optimum trouvé → Sortie de l'algorithme

Sinon
*Règle (3)*

Il n'ya pas de passage par zéro de la dérivée seconde entre $X_L$ et $X_R$. On va alors approximer l'optimum par le point pour lequel la valeur de la dérivée seconde est minimale (la plus proche de zéro) en valeur absolue. La détermination selon cette règle se fait par les étapes suivantes :

- $DAV_{opt} = X_L$, $E_m = Abs(E_L)$;
- Si ($Abs(E_C) < E_m$) alors $OAV = X_C$ et $E_m = Abs(E_C)$
- Si ($Abs(E_R) < E_m$) alors $OAV = X_R$
- Optimum approché → Sortie de l'algorithme

À la sortie de l'algorithme, l'une des trois règles a été nécessairement appliquée, et la valeur $DAV_{opt}$ trouvée pour le DAV optimal est comprise entre $X_L$ et $X_R$.

Par sécurité, on peut prévoir une condition supplémentaire pour empêcher toute modification du DAV dans le cas où les mesures du PEA ne donnent pas une caractéristique en forme de sigmoïde décroissante dans le sens des DAV croissants. On peut par exemple appliquer une condition $Y_2 > Y_4$, ce qui revient à tester que la pente de la courbe PEA = f(DAV) autour du point central $X_C$ est bien négative.

*Détection d'activité du patient*

Selon un autre aspect de l'invention, l'algorithme d'optimisation du DAV n'est pas appliqué de façon systématique, mais seulement sur détection d'une variation significative de l'activité du patient (passage du repos à l'effort et *vice versa*), car c'est dans ce cas précisément qu'il est nécessaire d'adapter ou d'asservir le DAV si l'on veut améliorer en permanence l'état hémodynamique du patient.

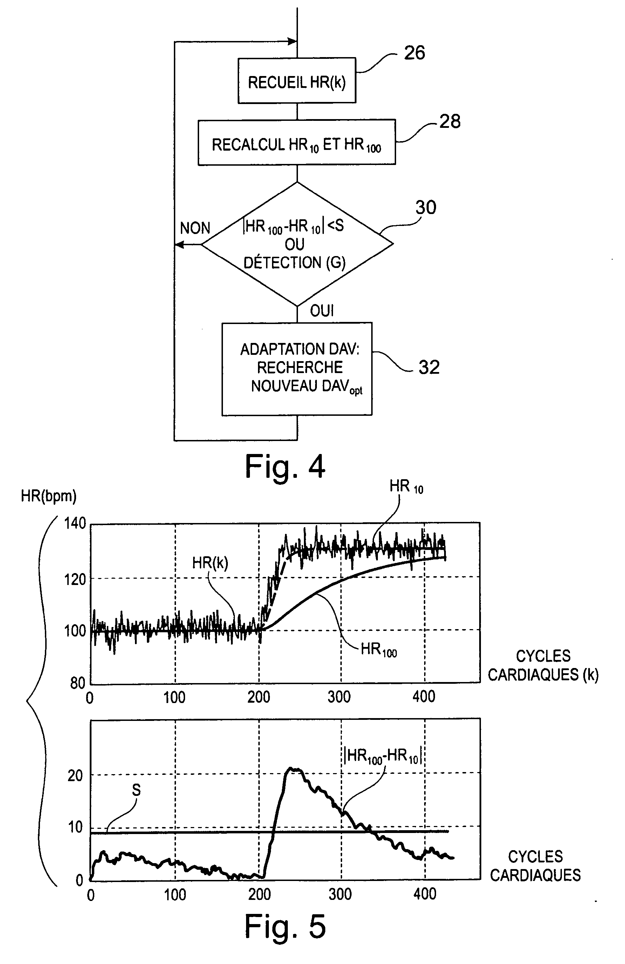

On va décrire en référence aux Figures 4 et 5 un algorithme spécifiquement adapté de détection d'un changement de l'état hémodynamique du patient par passage du repos à l'effort ou *vice versa*.

Comme illustré sur l'organigramme de la Figure 4, on recueille en permanence la valeur HR(k) du rythme cardiaque, à chaque cycle cardiaque k (bloc 26). Puis on calcule ou on met à jour une moyenne à court terme $HR_{10}$ et une moyenne à long terme $HR_{100}$ du rythme cardiaque, respectivement sur les dix derniers ou les cent derniers cycles cardiaques :

$$HR_{10} = (1-1/_{10})HR_{10} + HR(k)/_{10}$$
$$HR_{100} = (1-1/_{100})*HR_{100} + HR(k)/_{100}$$

L'étape suivante (bloc 30) consiste à tester une variation rapide du rythme cardiaque, représentative très vraisemblablement d'un changement du niveau d'activité du patient.

Pour cela, on calcule la différence $|(HR_{100} - HR_{10})|$ entre les moyennes $HR_{100}$ et $HR_{10}$ et on compare (test 30) cette différence à un seuil S. Si la condition $|(HR_{100} - HR_{10})| > S$ est vérifiée, alors l'algorithme de recherche d'un nouveau DAV optimum est déclenché (bloc 32).

On notera que, en variante ou en complément, le test 30 conduisant au déclenchement de l'algorithme peut être conditionné à une autre technique de détection de l'effort, notamment au signal délivré par un accéléromètre G (capteur 24 sur la Figure 1).

La Figure 5 illustre sur le chronogramme du haut, les variations des trois paramètres HR(k), $HR_{10}$ et $HR_{100}$ au cours du temps, avec un exemple d'augmentation brusque du niveau d'effort du patient à partir du cycle cardiaque n° 200.

Cette variation brusque se répercute, comme illustré sur le chronogramme du bas de la Figure 5, par le franchissement du seuil S par la différence $|(HR_{100} - HR_{10})|$, ce qui permet de déclencher l'adaptation du DAV à l'effort avec un très faible retard.

**Revendications**

**1.** Un dispositif médical actif, comprenant

- une prothèse cardiaque implantable de type stimulateur, défibrillateur et/ou resynchroniseur, comprenant :

- • des moyens de détection d'événements auriculaires et ventriculaires,
- • des moyens de stimulation ventriculaire, et
- • des moyens pour appliquer aux moyens de stimulation un délai atrioventriculaire DAV, compté à partir de la détection d'un événement auriculaire spontané ou stimulé et à l'issue duquel une stimulation ventriculaire est appliquée en l'absence de détection d'un événement ventriculaire spontané correspondant ;

- un capteur hémodynamique
- des moyens d'analyse du signal délivré par le capteur hémodynamique, aptes à délivrer un paramètre hémodynamique dont la variation en fonction du DAV est une fonction représentée par une caractéristique de type sigmoïde ; et
- des moyens de recherche d'un DAV optimal par analyse de ladite caractéristique de type sigmoïde,

**caractérisé :**

- **en ce que** les moyens de recherche d'un DAV optimal sont des moyens opérant par tests effectués sur un nombre limité de valeurs discrètes de DAV et déduction de la position de l'optimum directement à partir des résultats de ces tests,
- et **en ce que** ces moyens comprennent:

- • des moyens pour appliquer un DAV de référence ($X_c$) constitué par la valeur courante du DAV optimal ou à défaut une valeur predéterminée de DAV, et mesurer le paramètre hémodynamique correspondant (Y3) ;
- • des moyens, pour appliquer au moins un DAV ($X_L$, $X_{LL}$) inférieur au DAV de référence, désigné DAV à gauche, et mesurer le paramètre hémodynamique correspondant ($Y_1$, $Y_2$) ;
- • des moyens pour appliquer au moins un DAV ($X_R$, $X_{RR}$) supérieur au DAV de référence, désigné DAV à droite, et mesurer le paramètre hémodynamique correspondant ($Y_4$, $Y_5$) ;

• des moyens pour évaluer la dérivée seconde de ladite fonction aux points respectifs ($X_C$,$Y_3$ ; $X_L$,$Y_2$ ; $X_R$,$Y_4$) de la caractéristique correspondant au DAV de référence, au DAV à gauche et au DAV à droite ;

• des moyens pour estimer, à partir des valeurs des dérivées secondes ainsi évaluées, la position d'un point intermédiaire de la caractéristique pour lequel la dérivée seconde présente une valeur nulle ou minimale ; et

• des moyens pour désigner le DAV correspondant à ce point intermédiaire comme DAV optimal.

2. Le dispositif de la revendication 1, comprenant :

- des moyens pour appliquer deux DAV à gauche ($X_L$, $X_{LL}$), respectivement inférieurs au DAV de référence ($X_C$) de deux intervalles de temps différents;

- des moyens pour appliquer deux DAV à droite ($X_R$, $X_{RR}$), respectivement supérieurs au DAV de référence ($X_C$) de deux intervalles de temps différents ;

et dans lequel :

- les moyens pour estimer la dérivée seconde de la fonction au point correspondant au DAV de référence sont des moyens opérant par calcul d'accroissement de la fonction entre le DAV à gauche ($X_L$) et le DAV à droite ($X_R$) encadrant le DAV de référence ;

- les moyens pour estimer la dérivée seconde de la fonction au point correspondant au DAV à gauche sont des moyens opérant par calcul d'accroissement entre les deux DAV à gauche ($X_L$, $X_{LL}$) ; et

- les moyens pour estimer la dérivée seconde de la fonction au point correspondant au DAV à droite sont des moyens opérant par calcul d'accroissement entre les deux DAV à droite ($X_R$, $X_{RR}$).

3. Le dispositif de la revendication 1, dans lequel les valeurs consécutives ($X_{LL}$, $X_L$, $X_C$, $X_R$, $X_{RR}$) desdits DAV à gauche, DAV à droite et DAV de référence sont séparées par des pas temporels ($D_1$, $D_2$, $D_3$, $D_4$) égaux.

4. Le dispositif de la revendication 1, dans lequel les moyens pour estimer la position du point intermédiaire comprennent :

- des moyens pour déterminer l'existence d'un passage par zéro entre les valeurs de dérivée seconde correspondant au DAV à gauche et au DAV à droite ; et

- des moyens pour, dans ce cas, opérer un calcul d'interpolation linéaire entre les points correspondant au DAV à gauche et au DAV à droite.

5. Le dispositif de la revendication 1, dans lequel les moyens pour estimer la position du point intermédiaire comprennent :

- des moyens pour déterminer l'absence de passage par zéro entre les valeurs de dérivée seconde correspondant au DAV à gauche et au DAV à droite ; et

- des moyens pour, dans ce cas, sélectionner comme point intermédiaire, parmi les trois points correspondant au DAV de référence, au DAV à gauche et au DAV à droite, celui minimisant la valeur de dérivée seconde.

6. Le dispositif de la revendication 1, comprenant en outre :

- des moyens pour déterminer le signe de la pente de la caractéristique au point correspondant au DAV de référence ; et

- des moyens pour inhiber les moyens de recherche d'un DAV optimal en cas de détection d'une pente de signe non conforme.

7. Le dispositif de la revendication 1, comprenant en outre des moyens d'analyse de l'état d'activité du patient, aptes à conditionner la mise en oeuvre des moyens de recherche d'un DAV optimal à la détection d'un changement d'activité du patient.

8. Le dispositif de la revendication 1, dans lequel les moyens d'analyse de l'état d'activité comprennent des moyens aptes à comparer une moyenne à court terme ($HR_{10}$) et une moyenne à long terme ($HR_{100}$) de la fréquence cardiaque (HR), et à activer les moyens de recherche d'un DAV optimal lorsque l'écart entre ces deux moyennes franchit un seuil (S) prédéterminé.

9. Le dispositif de la revendication 1, dans lequel le capteur hémodynamique est un capteur d'accélération endocardiaque EA, et le paramètre hémodynamique est une valeur de pic d'accélération endocardiaque PEA, dérivée de l'un et/ou l'autre des deux pics de l'accélération endocardiaque PEA1 et/ou PEA2 apparaissant respectivement lors de la phase de contraction ventriculaire isovolumique et lors de la phase de relaxation ventriculaire isovolumique.

## Claims

1. Active medical device, comprising:

   - an implantable cardiac prosthesis of the pacemaker, defibrillator and/or resynchronizer type, comprising:

     • means for detection of atrial and ventricular events,
     • means for ventricular stimulation, and
     • means for applying to the means for stimulation an atrioventricular delay AVD, which is counted from the moment of detection of a spontaneous or stimulated atrial event and at the end of which a ventricular stimulation is applied in the absence of detection of a corresponding spontaneous ventricular event;

   - a haemodynamic sensor;
   - means for analysing the signal delivered by the haemodynamic sensor and for delivering a haemodynamic parameter whose variation as a function of the AVD is a function represented by a sigmoid characteristic; and
   - means for searching for an optimal AVD by analysing said sigmoid characteristic,

   **characterized:**

   - **in that** the means for searching for an optimal AVD are means operating by tests carried out on a limited number of discrete values of AVD and deduction of the position of the optimum directly from the results of these tests,
   - and **in that** these means comprise:

     • means for applying a reference AVD ($X_c$) formed by the current value of the optimal AVD or, failing this, a predetermined AVD value, and for measuring the corresponding haemodynamic parameter ($Y_3$) ;
     • means for applying at least one AVD ($X_L$, $X_{LL}$) lower than the reference AVD and designated left AVD, and for measuring the corresponding haemodynamic parameter ($Y_1$, $Y_2$);
     • means for applying at least one AVD ($X_R$, $X_{RR}$) greater than the reference AVD and designated right AVD, and for measuring the corresponding haemodynamic parameter ($Y_4$, $Y_5$);
     • means for evaluating the second derivative of said function at the respective points ($X_c$, $Y_3$; $X_L$, $Y_2$; $X_R$, $Y_4$) of the characteristic corresponding to the reference AVD, to the left AVD and to the right AVD;

     • means for estimating, from the values of second derivatives thus evaluated, the position of an intermediate point of the characteristic for which the second derivative has a zero or minimal value; and
     • means for designating the corresponding AVD at this intermediate point as the optimal AVD.

2. Device according to Claim 1, comprising:

   - means for applying two left AVD ($X_L$, $X_{LL}$), respectively lower than the reference AVD ($X_c$) by two different time intervals;
   - means for applying two right AVD ($X_R$, $X_{RR}$), respectively greater than the reference AVD ($X_c$) by two different time intervals;

   and in which

   - the means for estimating the second derivative of the function at the point corresponding to the reference AVD are means operating by computing increments of the function between the left AVD ($X_L$) and the right AVD ($X_R$) framing the reference AVD;
   - the means for estimating the second derivative of the function at the point corresponding to the left AVD are means operating by computing increments between the two left AVD ($X_L$, $X_{LL}$) : and
   - the means for estimating the second derivative of the function at the point corresponding to the right AVD are means operating by computing increments between the two right AVD ($X_R$, $X_{RR}$).

3. Device according to Claim 1, in which the consecutive values ($X_{LL}$, $X_L$, $X_C$, $X_R$, $X_{RR}$) of said left AVD, right AVD and reference AVD are separated by equal temporal steps ($D_1$, $D_2$, $D_3$, $D_4$).

4. Device according to Claim 1, in which the means for estimating the position of the intermediate point comprise:

   - means for determining the existence of a zero crossing between the second-derivative values corresponding to the left AVD and to the right AVD; and
   - means for calculating, in this case, a linear interpolation between the points corresponding to the left AVD and to the right AVD.

5. Device according to Claim 1, in which the means for estimating the position of the intermediate point comprise:

- means for determining the absence of a zero crossing between the second-derivative values corresponding to the left AVD and to the right AVD; and

- means for in this case selecting as an intermediate point, from the three points corresponding to the reference AVD, to the left AVD and to the right AVD, the one minimizing the second-derivative value.

6. Device according to Claim 1, further comprising:

- means for determining the sign of the slope of the characteristic at the point corresponding to the reference AVD; and
- means for inhibiting the means for searching for an optimal AVD in the event of defection of a slope of non-conforming sign.

7. Device according to Claim 1, further comprising means for analysing the state of activity of the patient and for initiating the means for searching for an optimal AVD in response to the detection of a change in the patient's activity.

8. Device according to Claim 1, in which the means for analysing the state of activity comprise means for comparing a short-term heart rate average ($HR_{10}$) and a long-term heart rate average ($HR_{100}$) and for initiating the means for searching for an optimal AVD when the difference between these two averages exceeds a predetermined threshold (S).

9. Device according to Claim 1, in which the haemodynamic sensor is a sensor of endocardial acceleration EA, and the haemodynamic parameter is a peak endocardial acceleration PEA value, derived from at least one of the two endocardial acceleration peaks PEAL and PEA2 which appear, respectively, during the isovolumetric ventricular contraction phase and during the isovolumetric ventricular relaxation phase.

**Patentansprüche**

1. Aktive medizinische Vorrichtung, die enthält:

- eine implantierbare Herzprothese von der Art Schrittmacher, Defibrillator und/oder Resynchronisierer, die enthält:

• Einrichtungen zur Erfassung aurikulärer und ventrikulärer Ereignisse,
• Einrichtungen zur ventrikulären Stimulation, und
• Einrichtungen zur Anwendung einer atrioventrikulären Verzögerung DAV an die Stimulationseinrichtungen, gezählt ab der Erfassung eines spontanen oder stimulierten aurikulären Ereignisses, und nach der eine ventrikuläre Simulation in Abwesenheit der Erfassung eines entsprechenden spontanen ventrikulären Ereignisses angewendet wird;

- einen hämodynamischen Sensor;
- Einrichtungen zur Analyse des vom hämodynamischen Sensor gelieferten Signals, die einen hämodynamischen Parameter liefern können, dessen Änderung abhängig von der DAV eine durch ein Merkmal vom sigmoidalen Typ dargestellte Funktion ist; und
- Einrichtungen zur Suche einer optimalen DAV durch Analyse des Merkmals vom sigmoidalen Typ,

**dadurch gekennzeichnet, dass**:

- die Einrichtungen zur Suche einer optimalen DAV Einrichtungen sind, die durch Tests, die an einer begrenzten Anzahl von diskreten DAV-Werten durchgeführt werden, und durch Ableitung der Position des Optimums direkt ausgehend von den Ergebnissen dieser Tests arbeiten,
- und dass diese Einrichtungen enthalten:

• Einrichtungen, um eine Bezugs-DAV ($X_C$), die vom laufenden Wert der optimalen DAV oder sonst einem vorbestimmten DAV-Wert gebildet wird, anzuwenden und den entsprechenden hämodynamischen Parameter ($Y_3$) zu messen;
• Einrichtungen, um mindestens eine DAV ($X_L$, $X_{LL}$) niedriger als die Bezugs-DAV anzuwenden, als linke DAV bezeichnet, und den entsprechenden hämodynamischen Parameter ($Y_1$, $Y_2$) zu messen;
• Einrichtungen, um mindestens eine DAV ($X_R$, $X_{RR}$) höher als die Bezugs-DAV anzuwenden, als rechte DAV bezeichnet, und den entsprechenden hämodynamischen Parameter ($Y_4$, $Y_5$) zu messen;
• Einrichtungen, um die zweite Ableitung der Funktion an den jeweiligen Punkten ($X_C$, $Y_3$; $X_L$, $Y_2$; $X_R$, $Y_4$) des der Bezugs-DAV, der linken DAV und der rechten DAV entsprechenden Merkmals zu ermitteln;
• Einrichtungen, um ausgehend von den Werten der so ermittelten zweiten Ableitungen die Position eines Zwischenpunkts des Merkmals zu schätzen, für den die zweite Ableitung einen Wert Null oder einen minimalen Wert hat; und
• Einrichtungen, um die diesem Zwischenpunkt entsprechende DAV als optimale DAV zu bezeichnen.

2. Vorrichtung nach Anspruch 1, die enthält:

- Einrichtungen zur Anwendung von zwei linken DAV ($X_L$, $X_{LL}$), die jeweils niedriger sind als die Bezugs-DAV ($X_C$) von zwei unterschiedlichen Zeitintervallen;
- Einrichtungen zur Anwendung von zwei rechten DAV ($X_R$, $X_{RR}$), die jeweils höher sind als die Bezugs-DAV ($X_C$) von zwei unterschiedlichen Zeitintervallen;

und bei der:

- die Einrichtungen zur Schätzung der zweiten Ableitung der Funktion am der Bezugs-DAV entsprechenden Punkt Einrichtungen sind, die durch Berechnung der Zunahme der Funktion zwischen der linken DAV ($X_L$) und der rechten DAV ($X_R$) arbeiten, die die Bezugs-DAV umrahmen;
- die Einrichtungen zur Schätzung der zweiten Ableitung der Funktion am der linken DAV entsprechenden Punkt Einrichtungen sind, die durch Berechnung der Zunahme zwischen den zwei linken DAV ($X_L$, $X_{LL}$) arbeiten; und
- die Einrichtungen zur Schätzung der zweiten Ableitung der Funktion am der rechten DAV entsprechenden Punkt Einrichtungen sind, die durch Berechnung der Zunahme zwischen den zwei rechten DAV ($X_R$, $X_{RR}$) arbeiten.

3. Vorrichtung nach Anspruch 1, bei der die aufeinanderfolgenden Werte ($X_{LL}$, $X_L$, $X_C$, $X_R$, $X_{RR}$) der linken DAV, der rechten DAV und der Bezugs-DAV durch gleiche Zeitschritte ($D_1$, $D_2$, $D_3$, $D_4$) getrennt sind.

4. Vorrichtung nach Anspruch 1, bei der die Einrichtungen zur Schätzung der Position des Zwischenpunkts enthalten:

- Einrichtungen zur Bestimmung des Vorhandenseins eines Nulldurchgangs zwischen den Werten zweiter Ableitung, die der linken DAV und der rechten DAV entsprechen; und
- Einrichtungen, um in diesem Fall eine Berechnung einer linearen Interpolation zwischen den der linken DAV und der rechten DAV entsprechenden Punkten durchzuführen.

5. Vorrichtung nach Anspruch 1, bei der die Einrichtungen zum Schätzen der Position des Zwischenpunkts enthalten:

- Einrichtungen zur Bestimmung der Abwesenheit eines Nulldurchgangs zwischen den Werten zweiter Ableitung entsprechend der linken DAV und der rechten DAV; und
- Einrichtungen, um in diesem Fall als Zwischenpunkt unter den drei der Bezugs-DAV, der linken DAV und der rechten DAV entsprechenden Punkten denjenigen auszuwählen, der den Wert zweiter Ableitung minimiert.

6. Vorrichtung nach Anspruch 1, die außerdem enthält:

- Einrichtungen zur Bestimmung des Vorzeichens der Steigung des Merkmals am der Bezugs-DAV entsprechenden Punkt; und
- Einrichtungen zum Sperren der Einrichtungen zur Suche einer optimalen DAV im Fall der Erfassung einer Steigung mit nicht konformem Vorzeichen.

7. Vorrichtung nach Anspruch 1, die außerdem Einrichtungen zur Analyse des Aktivitätszustands des Patienten enthält, die in der Lage sind, die Anwendung der Einrichtungen zur Suche einer optimalen DAV bei Erfassung einer Aktivitätsänderung des Patienten zu beeinflussen.

8. Vorrichtung nach Anspruch 1, bei der die Einrichtungen zur Analyse des Aktivitätszustands Einrichtungen enthalten, die in der Lage sind, einen kurzfristigen Mittelwert ($HR_{10}$) und einen langfristigen Mittelwert ($HR_{100}$) der Herzfrequenz (HR) zu vergleichen, und die Einrichtungen zur Suche einer optimalen DAV zu aktivieren, wenn die Abweichung zwischen diesen zwei Mittelwerten eine vorbestimmte Schwelle (S) überschreitet.

9. Vorrichtung nach Anspruch 1, bei der der hämodynamische Sensor ein Sensor der endokardialen Beschleunigung EA ist, und der hämodynamische Parameter ein Spitzenwert der endokardialen Beschleunigung PEA ist, abgeleitet von der einen und/oder anderen der zwei endokardialen Beschleunigungsspitzen PEA1 und/oder PEA2, die je in der Phase der isovolumetrischen ventrikulären Kontraktion bzw. in der Phase der isovolumetrischen ventrikulären Relaxation auftreten.

## Fig. 1

## Fig. 2

Fig. 3

Fig. 4

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2070562 A1
- WO 2006090397 A2
- WO 2006126185 A2
- WO 2008010220 A
- EP 0515319 A1
- EP 0655260 A1

**Littérature non-brevet citée dans la description**

- **JM DUPUIS et al.** *Programming Optimal Atrioventricular Delay in Dual Chamber Pacing Using Peak Endocardial Acceleration : Comparison with a Standard Echocardiographic Procedure, PACE,* 2003, vol. 26, 210-213